# FASCICULE DE BREVET EUROPEEN

(11) **EP 2 459 527 B1**
(45) Date de publication et mention de la délivrance du brevet: **07.12.2016**
(21) Numéro de dépôt: 10763202.8
(22) Date de dépôt: 29.07.2010
(51) Int. Cl.: C07C 315/06, C07C 317/04, C07C 319/26, C07C 321/14

(54) **COMPOSITION DE SOLVANT À BASE D'OXYDE DE SULFURE ORGANIQUE À ODEUR MASQUÉE**
LÖSEMITTELZUSAMMENSETZUNG BASIEREND AUF EINEM OXID EINES ORGANISCHEN SULFIDS MIT MASKIERTEM GERUCH
SOLVENT COMPOSITION BASED ON AN OXIDE OF AN ORGANIC SULFIDE WITH MASKED ODOUR

(30) Priorité: 31.07.2009 FR 0955398; 18.08.2009 US 234680 P; 12.01.2010 FR 1050157
(43) Date de publication de la demande: 06.06.2012
(73) Titulaire: Arkema France, 92700 Colombes (FR)
(72) Inventeur: SCHMITT, Paul-Guillaume, F-64230 Lescar (FR); MONGUILLON, Bernard, 64210 ARBONNE (FR); VAUTHRIN, Mélanie, F-64230 Denguin (FR)
(74) Mandataire: Lhoste, Catherine
(86) Numéro de dépôt international: PCT/FR2010/051615
(87) Numéro de publication internationale: WO 2011/012820

(56) Documents cités:
- EP-A1- 0 976 726
- US-A- 6 042 640
- US-A1- 2009 005 601

## Description

La présente invention concerne le domaine des sulfures organiques et plus particulièrement celui des oxydes de sulfures d'alkyle ou de dialkyle, et notamment du diméthylsulfoxyde (ou DMSO).

Il est bien connu que les sulfures organiques présentent en général une odeur forte, désagréable, voire agressive. Les oxydes de sulfures organiques, en particulier le DMSO, peuvent présenter une odeur moins agressive, mais toutefois, suivant les concentrations en impuretés, cette odeur peut être désagréable, et gênante pour l'utilisateur final.

Jusqu'à aujourd'hui, cet inconvénient ne constitue pas un réel problème puisque les oxydes de sulfures organiques, et en particulier le DMSO, sont généralement utilisés en faibles quantités, le plus souvent dans des compositions, par exemple pharmaceutiques, cosmétiques, phytosanitaires et autres. De telles compositions comprennent nombre d'autres composants, bien souvent beaucoup plus odorants, voire malodorants, dont les odeurs sont couramment masquées par des solvants, des arômes, des parfums et autres. Ainsi, les inconvénients liés à l'odeur des oxydes de sulfures organiques n'ont jusqu'à aujourd'hui pas constitué un réel problème auquel l'homme du métier a pu être confronté.

Cependant, les oxydes de sulfures organiques, et en particulier le DMSO, sont aujourd'hui susceptibles de trouver d'autres utilisations dans lesquelles leurs odeurs pourraient représenter un frein à leur développement. En effet, en raison du remplacement programmé des solvants toxiques, tels que par exemple la N-méthylpyrrolidone (NMP), le N,N-diméthylformamide (DMF) ou le chlorure de méthylène, les oxydes de sulfures organiques, et le DMSO en particulier, représentent des solvants de choix en raison de leurs propriétés que sont leur faible toxicité, et leur fort pouvoir solvant.

Pour l'utilisation des oxydes de sulfures organiques en tant que solvant, il reste donc à solutionner le problème des odeurs inhérentes à ces produits. Le document US 6 042 640 décrit un procédé de désodorisation de solvants sulfurés, par exemple DMSO, par oxydation des impuretés sulfures. La Demanderesse a maintenant découvert qu'il est possible de masquer, d'odoriser, les oxydes de sulfures organiques utilisés en tant que solvants afin de permettre leurs utilisations sans être incommodés par les odeurs intrinsèques desdits oxydes, tout en conservant les propriétés de solvant propres auxdits oxydes de sulfures organiques.

Ainsi l'invention a pour objet une composition de solvant comprenant :
a) au moins 50% et de préférence au moins 70%, de manière tout à fait préférée au moins 80% en poids d'au moins un oxyde de sulfure organique de formule générale (1) : 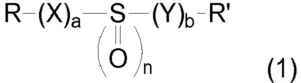 dans laquelle
   - X et Y, identiques ou différents, sont choisis, indépendamment l'un de l'autre, parmi oxygène, soufre, -SO-, -SO₂-, -NH-, et -NR"- ;
   - a et b, identiques ou différents, représentent, indépendamment l'un de l'autre, 0 ou 1 ; n est égal à 1 ou 2 ;
   - R, R' et R", identiques ou différents, sont choisis indépendamment les uns des autres, parmi un radical alkyle, linéaire ou ramifié, contenant de 1 à 12 atomes de carbone, un radical alcényle, linéaire ou ramifié, contenant de 2 à 12 atomes de carbone, et un radical aryle contenant de 6 à 10 atomes de carbone ; R, R' et R" pouvant éventuellement être substitués par des radicaux choisis parmi alkyle, alcényle, aryle, halogène, et pouvant éventuellement contenir un ou plusieurs hétéroatomes choisis parmi O, S, N, P et Si ; R et R' pouvant en outre former ensemble et avec les atomes qui les portent, une structure cyclique hydrocarbonée et contenant éventuellement un ou plusieurs hétéroatomes choisis parmi O, S et N, ladite structure cyclique comportant au total 5, 6, 7, 8 ou 9 sommets ; et
b) de 10 ppm, à 2%, de préférence de 10 ppm à 1% en poids par rapport au poids total de la composition, d'au moins un agent masquant d'odeur comprenant au moins un composé choisi parmi les mono-esters, les di- et/ou tri-esters, les alcools, les aldéhydes, les cétones et les terpènes.

La quantité d'agent masquant (composition b)) peut varier dans de grandes proportions dans la plage indiquée ci-dessus, selon l'effet souhaité, l'intensité de l'odeur à masquer, les teneurs résiduelles respectives des diverses impuretés pouvant être présentes dans le(s) composant(s) a) précédemment définis, et autres.

Des quantités d'agent masquant inférieures à quelques ppm peuvent être trop faibles pour obtenir l'effet souhaité. Des quantités d'agent masquant supérieures à 2% peuvent avoir des effets néfastes selon les applications visées pour les oxydes de sulfures organiques en tant que solvants.

De préférence, et de manière non limitative, la teneur en agent(s) masquant d'odeur b) est comprise entre 0,001 % et 0,2% en poids par rapport au poids total de la composition, de préférence comprise entre 100 ppm et 1000 ppm, par exemple 500 ppm en poids.

Dans la description de la présente invention, les pourcentages sont indiqués en poids, sauf mention spécifique contraire. Sauf mention contraire, « ppm » signifie partie par million en poids. Par « radical aryle », on entend un radical hydrocarboné aromatique, choisi de préférence parmi phényle et naphtyle. De préférence le radical aryle est un radical phényle.

Selon un aspect préféré de la présente invention, le composant a) répond à la formule (1) dans laquelle a est 0 et -(Y)_{b}- représente -(S)ₓ-, où x représente 0 ou 1, de préférence 0. On préfère également les composants a) de formule (1) pour lesquels les radicaux R et R' sont identiques et sont choisis parmi un radical alkyle linéaire ou ramifié comportant de 1 à 12 atomes de carbone, de préférence de 1 à 6 atomes de carbone, de préférence encore de 1 à 4 atomes de carbone, un radical alcényle linéaire ou ramifié comportant de 2 à 12 atomes de carbone, de préférence de 2 à 6 atomes de carbone, de préférence encore de 2 à 4 atomes de carbone, et un radical aryle, de préférence phényle.

Selon un autre aspect préféré, parmi les composants a) de formule (1) ci-dessus, on préfère ceux pour lesquels a et b représentent chacun 1 et X et Y sont choisis indépendamment parmi oxygène, soufre, -NH-, et -NR"-.

Selon un mode de réalisation, le composant a) utilisé dans la composition selon la présente invention est un oxyde de sulfure organique, obtenu selon tout procédé connu en soi, ou encore disponible dans le commerce, et de préférence à teneur réduite en impuretés volatiles. De telles impuretés sont par exemple, et notamment lorsque le composé a) est le DMSO, le sulfure de diméthyle (DMS), le diméthyldisulfure (DMDS) et/ou le bis(méthylthio)méthane, également connu sous le nom de 2,4-dithiapentane (BMTM).

Toute méthode connue de l'homme du métier destinée à éliminer, ou tout au moins réduire, les impuretés volatiles précitées peut convenir, parmi lesquelles on peut citer, de manière non limitative la distillation, la cristallisation, l'évaporation sous courant de gaz inerte tel que azote, air, et autres.

Dans le cas du DMSO, les teneurs en impuretés, telles DMS, DMDS et/ou BMTM, doivent avantageusement être inférieures 100 ppm, de préférence inférieures à 50 ppm, de préférence encore inférieures à 10 ppm, pour chacune des impuretés prises séparément.

Selon un mode de réalisation préféré, le composant a) de la composition selon la présente invention répond à la formule (1') : 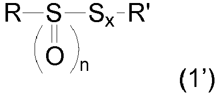 dans laquelle R est choisi parmi un radical alkyle, linéaire ou ramifié, contenant de 1 à 4 atomes de carbone, un radical alcényle, linéaire ou ramifié, contenant de 2 à 4 atomes de carbone, et un radical aryle, de préférence phényle, n est égal à 1 ou 2 ; x représente 0 ou 1 ; R' est choisi parmi un radical alkyle, linéaire ou ramifié, contenant de 1 à 4 atomes de carbone, un radical alcénylène, linéaire ou ramifié, contenant de 2 à 4 atomes de carbone, et un radical aryle, de préférence phényle.

Selon un mode de réalisation particulièrement préféré, le composant a) de la composition selon la présente invention répond à la formule (1a) : 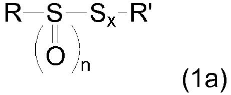 dans laquelle R et R', identiques ou différents, sont choisis parmi un radical alkyle, linéaire ou ramifié, contenant de 1 à 4 atomes de carbone, un radical alcényle, linéaire ou ramifié, contenant de 2 à 4 atomes de carbone, et un radical phényle ; n est égal à 1 ou 2 ; et x est égal à 0.

De préférence le composant a) de formule (1a) est le DMSO.

Selon l'invention, le masquage de l'odeur de l'oxyde de sulfure organique répondant à la formule (1), (1') ou (1a) précédemment décrites, est obtenu, par ajout audit oxyde, une composition définie sous b) précédemment.

La présente invention présente l'avantage de masquer l'odeur désagréable d'au moins un oxyde de sulfure organique, sans en modifier chimiquement la nature. Ainsi, la présente invention propose une composition comprenant a) une quantité majoritaire d'au moins un oxyde de sulfure organique de formule (1), (1') ou (1a) définies précédemment, à laquelle est ajoutée une quantité minoritaire d'une composition b) masquant l'odeur désagréable du composant a).

La composition à odeur masquée selon la présente invention peut être préparée selon tout procédé connu en soi en combinant simplement au moins un composant a) avec au moins une composition b) de masquage d'odeur. On peut par exemple ajouter au moins une composition b) à au moins un composant a), ou vice-versa, éventuellement sous agitation et/ou éventuellement en chauffant. Plus généralement, toute méthode connue de mélange et/ou de chauffage peut être utilisée.

La préparation de la composition selon l'invention peut par exemple être effectuée sous pression atmosphérique, à une température comprise entre 0°C et 100°C, de préférence comprise entre la température ambiante et environ 80°C. La préparation peut également être effectuée sous pression ou sous dépression, à des températures comprises dans les gammes indiquées ci-dessus.

La période de temps requise pour la préparation de la composition à odeur masquée selon l'invention varie selon la nature et la quantité du ou des composant(s) a) et de la ou des composition(s) b), mais aussi en fonction de la température et de la pression choisies. En règle générale, cette durée correspond à la durée nécessaire pour obtenir un mélange homogène et produisant l'effet recherché de masquage d'odeur du ou des composant(s) a) ; elle est généralement comprise entre quelques secondes et quelques minutes, voire une ou plusieurs heures.

Le procédé de préparation mentionné ci-dessus peut être réalisé par lots (procédé « batch ») ou encore en continu.

Comme indiqué précédemment, l'agent masquant d'odeur b) comprend un ou plusieurs des composés choisis parmi :
b1) les mono-esters ;
b2) les di- et/ou tri-esters ;
b3) les alcools, avantageusement les mono-alcools, comprenant de 1 à 30 atomes de carbone, de préférence de 6 à 20 atomes de carbone, de préférence encore de 8 à 11 atomes de carbone, lesdits atomes de carbone formant une chaîne linéaire ou ramifiée comportant éventuellement une ou plusieurs insaturation(s) sous forme de double(s) liaison(s), et comportant éventuellement une structure cyclique à 5 ou 6 chaînons, saturée, ou totalement ou partiellement insaturée ;
b4) les aldéhydes et/ou les cétones, en particulier les aldéhydes et/ou les cétones de formule R^{a}-CO-R^{b}, dans laquelle R^{a} représente une chaîne hydrocarbonée comportant de 1 à 6 atomes de carbone, linéaire ou ramifié, éventuellement comprenant une ou plusieurs insaturation(s) sous forme de double(s) liaison(s), et R^{b} représente l'atome d'hydrogène, une chaîne hydrocarbonée cyclique ou bien une chaîne hydrocarbonée linéaire ou ramifiée, éventuellement, mais de préférence, substituée par une structure cyclique, R^{b} comportant de 6 à 12 atomes de carbone, comprenant éventuellement une ou plusieurs insaturation(s) sous forme de double(s) liaison(s) et étant éventuellement substitué par un ou plusieurs groupes hydroxyle ; et
b5) les terpènes.

Comme exemples illustratifs, mais non limitatifs, de mono-esters mentionnés sous b1), on peut citer les esters d'acides en C₂-C₂₀ saturés ou insaturés, tels que les acétates, propionates, butyrates, méthylbutyrates, pentanoates, hexanoates, heptanoates, caproates, oléates, linoléates, linolénates, d'éthyle, de propyle, de butyle, de pentyle, de 2-méthylbutyle, d'*i*so-amyle, d'hexyle, de benzyle, de phényléthyle, de menthyle, de carvyle, et autres, ainsi que leurs mélanges.

Sont plus particulièrement préférés l'acétate d'*i*so-amyle, l'acétate d'hexyle, le butyrate de 2-méthylbutyle, le butyrate d'*i*so-amyle, l'acétate de benzyle, l'acétate de phényléthyle et les mélanges de ces composés.

Comme exemples illustratifs, mais non limitatifs, de di- et/ou tri-esters b2), on peut citer les *ortho*-phtalates, tel que l'*ortho*-phtalate de diéthyle ; les citrates, tels que le citrate de triéthyle ; et/ou les malonates, tel que le malonate de diéthyle.

Comme exemples illustratifs, mais non limitatifs, d'alcools b3) précédemment, on peut citer de préférence les mono-alcools, dont la fonction hydroxyle est de préférence portée par un atome de carbone sp². Il doit être entendu que la fonction hydroxyle peut également être portée par un atome de carbone inclus dans une structure cyclique comme définie précédemment.

Les alcools b3) pouvant être utilisés dans l'agent masquant d'odeur et tels que définis ci-dessus sont avantageusement et à titre d'exemples non limitatifs choisis parmi le menthol, le *néo*-menthol, l'alcool phényléthylique, l'alcool benzylique, le citronellol, le dihydromyrcénol, le dihydroterpinéol, le dimétol, l'éthyllinalol, le géraniol, le linalol, le tétrahydrolinalol, le tétrahydromyrcénol, le nérol, ainsi que les mélanges de deux ou plusieurs d'entre eux.

Comme exemples illustratifs, mais non limitatifs, d'aldéhydes et de cétones indiquées sous b4), on peut citer de préférence le propionaldéhyde, le butyraldéhyde, le valéraldéhyde, le capraldéhyde, le benzaldéhyde, le géranial, le néral, le citronellal, et de manière générale les aldéhydes ayant des groupements hydrocarbonés comportant une ou plusieurs insaturations de type oléfinique, la menthone, l'iso-menthone, le 1,8-cinéole, l'ascaridole, la flavonone, les damascones, les damascénones, les ionones, les irisones, les méthylionones, la frambinone (n° CAS 5471-51-2), ainsi que leurs mélanges de deux ou plusieurs d'entre eux en toutes proportions.

Comme exemples illustratifs, mais non limitatifs, de terpènes indiqués sous b5), on peut par exemple citer les terpinènes, le myrcène, le limonène, le terpinolène, les pinènes, le sabinène, le camphène, les mélanges de deux ou plusieurs d'entre eux, ainsi que les essences à base de terpènes, notamment celles comprenant ces ingrédients.

En outre, l'agent masquant d'odeur b) utilisable dans le cadre de la présente invention peut comprendre, en quantités minoritaires, d'autres agents (fragrances) habituellement utilisés dans le domaine de la parfumerie.

La composition b) destinée à masquer l'odeur des sulfures organiques, et telle que décrite précédemment peut, le cas échéant, voir si nécessaire, comprendre en outre un ou plusieurs additifs couramment utilisés dans le domaine. De tels additifs peuvent par exemple être choisis parmi, et de manière non limitative, les solvants, les pigments, les colorants, les conservateurs, les biocides, et autres.

Parmi les solvants, des exemples tout particulièrement préférés sont les alcools, les éthers, les esters et les glycols. De manière particulièrement avantageuse, le solvant est choisi parmi le phtalate de diéthyle, l'éthylène glycol, le propylène glycol, le di-éthylène glycol, le dipropylène glycol, les poly-éthylène glycols, les polypropylène glycols, et leurs mélanges, et de manière encore plus avantageuse parmi le phtalate de diéthyle, le dipropylène glycol, et leurs mélanges.

Il doit être compris que les mono-, di- ou tri-esters présents dans la composition b) d'agent masquant d'odeur, en tant que composant b1) et ou b2) peut également avoir les fonctions des solvants définis ci-dessus.

Selon un aspect préféré, l'agent masquant d'odeur utilisé dans la composition de la présente invention est choisi parmi les agents masquant d'odeurs qui comprennent :
- au moins un composant b1) ;
- au moins un composant b1) et au moins un composant b2) ;
- au moins un composant b1) et au moins un composant b3) ;
- au moins un composant b1) et au moins un composant b4) ;
- au moins un composant b1) et au moins un composant b5) ;
- au moins un composant b1), au moins un composant b2) et au moins un composant b3) ;
- au moins un composant b1), au moins un composant b2) et au moins un composant b4) ;
- au moins un composant b1), au moins un composant b2) et au moins un composant b5) ;
- au moins un composant b1), au moins un composant b3) et au moins un composant b4) ;
- au moins un composant b1), au moins un composant b3) et au moins un composant b5) ;
- au moins un composant b1), au moins un composant b2), au moins un composant b3) et au moins un composant b4) ;
- au moins un composant b1), au moins un composant b2), au moins un composant b3) et au moins un composant b5) ;
- au moins un composant b1), au moins un composant b2), au moins un composant b3), au moins un composant b4) et au moins un composant b5) ;
- au moins un composant b2) ;
- au moins un composant b2) et au moins un composant b3) ;
- au moins un composant b2) et au moins un composant b4) ;
- au moins un composant b2) et au moins un composant b5) ;
- au moins un composant b2), au moins un composant b3) et au moins un composant b4) ;
- au moins un composant b2), au moins un composant b3) et au moins un composant b5) ;
- au moins un composant b2), au moins un composant b4) et au moins un composant b5) ;
- au moins un composant b2), au moins un composant b3), au moins un composant b4) et au moins un composant b5) ;
- au moins un composant b3) ;
- au moins un composant b3) et au moins un composant b4) ;
- au moins un composant b3) et au moins un composant b5) ;
- au moins un composant b3), au moins un composant b4) et au moins un composant b5) ;
- au moins un composant b4) ;
- au moins un composant b4) et au moins un composant b5) ; et
- au moins un composant b5).

Selon un aspect encore plus préféré, l'agent masquant d'odeur utilisé dans la composition de la présente invention est choisi parmi les agents masquant d'odeurs qui comprennent :
- au moins un composant b1), au moins un composant b2) et au moins un composant b3) ;
- au moins un composant b1), au moins un composant b2) et au moins un composant b4) ;
- au moins un composant b1), au moins un composant b2) et au moins un composant b5) ;
- au moins un composant b1), au moins un composant b2), au moins un composant b3) et au moins un composant b4) ;
- au moins un composant b1), au moins un composant b2), au moins un composant b3) et au moins un composant b5) ; et
- au moins un composant b1), au moins un composant b2), au moins un composant b3), au moins un composant b4) et au moins un composant b5).

Comme indiqué précédemment, la composition selon la présente invention comprend au moins une composition d'agent masquant d'odeur b), ledit agent comprenant de 1% à 40%, de préférence de 2% à 35%, de préférence encore de 5% à 30% en poids, par rapport au poids total de la composition b), d'au moins un mono-ester mentionné sous b1).

La composition d'agent masquant d'odeur b) comprend également au moins un di- et/ou tri-ester b2), en une quantité allant de 10% à 70% en poids, de préférence de 15% à 65% en poids, de préférence encore de 20% à 60% en poids, par rapport au poids total de la composition b).

L'agent masquant d'odeur b) comprend également de 1% à 30%, de préférence de 5% à 25% en poids par rapport au poids total de la composition, d'au moins un alcool b3).

La quantité d'aldéhyde(s) ou de cétone(s) indiqué(s) sous b4) est avantageusement comprise dans une proportion allant de 0,5% à 20%, de préférence de 1% à 10% en poids par rapport au poids total de la composition.

L'agent masquant d'odeur peut éventuellement comprendre également jusqu'à 20%, de préférence de 1% à 10% en poids par rapport au poids total de la composition, d'au moins un terpène indiqué sous b5).

Selon un mode de réalisation préféré, l'agent masquant d'odeur b) comprend :
b1) de 1% à 40% en poids d'au moins un mono-ester ;
b2) de 10% à 70% en poids d'au moins un di- et/ou tri-ester ;
b3) de 1% à 30% en poids d'au moins un alcool ;
b4) de 0,5% à 20% en poids d'au moins un aldéhyde ou une cétone de formule R^{a}-CO-R^{b}, dans laquelle R^{a} représente une chaîne hydrocarbonée comportant de 1 à 6 atomes de carbone, linéaire ou ramifié, éventuellement comprenant une ou plusieurs insaturation(s) sous forme de double(s) liaison(s), et R^{b} représente l'atome d'hydrogène, une chaîne hydrocarbonée cyclique ou bien une chaîne hydrocarbonée linéaire ou ramifiée éventuellement mais de préférence substituée par une structure cyclique, R^{b} comportant de 6 à 12 atomes de carbone, comprenant éventuellement une ou plusieurs insaturation(s) sous forme de double(s) liaison(s) et étant éventuellement substitué par un ou plusieurs groupes hydroxyle ; et
b5) éventuellement jusqu'à 20% d'au moins un terpène.

Une composition typique d'agent masquant d'odeur adaptée pour les oxydes de sulfures organiques, selon la présente invention comprend en poids :
- de 5% à 30% en poids d'au moins un mono-ester b1), choisi parmi l'acétate d'*i*so-amyle, le méthyl-2-butyrate d'éthyle, le butyrate d'*i*so-amyle, l'acétate de phényléthyle, le caproate d'éthyle, l'acétate de benzyle, l'acétate d'hexyle et leurs mélanges ;
- de 20% à 60% en poids d'au moins un di- et/ou tri-ester b2) choisi(s) parmi les *ortho*-phtalates, tel que l'*ortho*-phtalate de diéthyle ; les citrates, tels que le citrate de triéthyle ; et les malonates, tel que le malonate de diéthyle, et leurs mélanges ;
- de 5% à 25% d'au moins un alcool, de préférence d'au moins deux alcools, de préférence encore d'au moins trois alcools, tels que décrits précédemment sous b3) ;
- de 1% à 10% d'au moins une cétone, de préférence au moins deux cétones, de préférence encore au moins trois cétones, telles que décrites précédemment sous b4) ; et
- de 1% à 10% d'au moins un, de préférence au moins deux, de préférence un mélange de, terpènes référencés plus haut sous b5).

Cette composition, notée C*ᵢ* dans la suite du présent exposé, est tout particulièrement adaptée pour le masquage de l'odeur, pour l'amélioration de l'odeur, des oxydes de sulfures organiques, et en particulier du DMSO.

Un exemple représentatif, mais non-limitatif, d'une telle composition C*ᵢ* est reproduite ci-dessous, dans laquelle chacun des composants comprend un, plusieurs, voire tous les composés listés :

| | |
|---|---|
| *Composant b1)* | 16,00% |
| comprenant acétate de benzyle, acétate d'hexyle, acétate d'*i*so-amyle, acétate de phényléthyle, caproate d'éthyle, méthyl-2-butyrate éthyle | |
| *Composant b3)* | |
| comprenant alcool phényléthylique, citronellol, géraniol, linalol, *cis*-3-hexènol | 20,60% |
| *Composant b4)* | |
| comprenant 1-(4-hydroxyphenyl) butane-3-one, *alpha*-irisone | 4,50% |
| *Composant b5)* | |
| terpènes d'orange | 7,00% |
| *Autres* | |
| comprenant citral, éthylmaltol, éthylméthylphénylglycidate | 1,90% |
| *Composant b2)* | |
| comprenant malonate de diéthyle, phtalate de diéthyle | q.s.p. 100,00% |

Ces compositions d'agents masquants pour les oxydes de sulfures organiques utilisés en tant que solvant sont données à titre d'exemples et n'ont rien de restrictif quant à la diversité potentielle de compositions que permet la présente invention définie à l'aide des revendications annexées.

Selon un autre aspect, la présente invention concerne une composition de solvant comprenant :
a) de 50% à 95%, de préférence de 70% à 90%, de manière tout à fait préférée 80% en poids d'au moins un oxyde de sulfure organique de formule générale (1) telle que définie précédemment ;
b) de 10 ppm, à 2%, de préférence de 10 ppm à 1% en poids par rapport au poids total de la composition, d'au moins un agent masquant d'odeur b) tel que défini précédemment ; et
c) de 5% à 50%, de préférence de 10% à 30%, de manière tout à fait préférée 20% en poids d'au moins un co-solvant.

Le co-solvant est typiquement choisi parmi les composés solubles au moins partiellement ou totalement dans les oxydes de sulfures organiques a), et en particulier dans le DMSO.

Ainsi, le co-solvant est de préférence choisi parmi les carbonates, esters, cétones, amines, amides, et les alcools, de préférence encore parmi les alcools, de préférence saturés, de préférence comportant de 1 à 10 atomes de carbone, et, par exemple et de manière non limitative, choisi parmi le méthanol, l'éthanol, le propanol, le butanol, le pentanol, l'éthylène-glycol, le propylène-glycol et le glycérol (propane-1,2,3-triol), de préférence parmi le méthanol, l'éthanol, l'éthylène-glycol et le glycérol. Un co-solvant préféré est le glycérol.

Une composition tout particulièrement préférée selon la présente invention comprend :
a) de 50% à 95%, de préférence de 70% à 90%, de manière tout à fait préférée 80% en poids d'au moins un oxyde de sulfure organique de formule générale (1), (1') ou (1a) telle que définie précédemment, et de préférence le DMSO ;
b) de 10 ppm, à 2%, de préférence de 10 ppm à 1% en poids par rapport au poids total de la composition, d'au moins un agent masquant d'odeur b) tel que défini précédemment, de préférence l'agent masquant C*ᵢ* ; et
c) de 5% à 50%, de préférence de 10% à 30%, de manière tout à fait préférée 20% en poids d'un alcool, de préférence le glycérol.

Par exemple, une composition selon la présente invention comprend :
a) 80% en poids de DMSO ;
b) de 10 ppm, à 2%, de préférence de 10 ppm à 1% en poids par rapport au poids total de la composition, de l'agent masquant C*ᵢ* ; et
c) 20% en poids de glycérol.

Les compositions de solvant selon la présente invention comprenant majoritairement au moins un oxyde de sulfure organique et au moins un agent masquant d'odeur trouvent des utilisations tout à fait avantageuses dans de nombreux domaines, où de tels solvants sont peu ou pas employés, en raison notamment de leur odeur.

De telles utilisations désormais possibles sont par exemples l'utilisation de solvant à base majoritairement d'oxyde(s) de sulfure(s) organique(s), et en particulier de DMSO, comme solutions nettoyantes ou décapantes (pour les peintures, dans le domaine de l'électronique, pour le décapage des photorésists, et autres), formulations agrochimiques, en particuliers formulations pesticides (herbicides, insecticides, fongicides, bactéricides, nématocides et autres), formulations dégivrantes, compositions pour liquides de refroidissement et autres formulations pour la synthèse ou la solubilisation (nettoyage ou mise en forme) de résines et/ou de polymères, parmi lesquels on peut citer poly-acrylates, polyméthacrylates, poly-acrylonitrile, poly(acétate de vinyle), poly-imides (telles que par exemple les poly-esters-imides, les poly-amides-imides, les poly-éthers-imides et les poly(amino-bis maléimides), polysulfones, polyesters, poly(alcools vinyliques), polyéthersulfones, polyamides, polyuréthanes, élastomères (tels que par exemple les EPDM, les SBR), résines époxy, résines phénoplast, résines aminoplast, polymères chlorés (tels que le PVC), polymères fluorés (tels que le PTFE, et le PVDF, en particulier les différents grades de Kynar^{®}, commercialisés par la société ARKEMA, et notamment le Kynar Flex^{®}), pour ne citer que quelques unes des utilisations possibles, sans être limitatif.

Les exemples suivants illustrent l'invention sans la limiter.

### Exemple 1 : Composition à base de DMSO à odeur masquée

Afin de caractériser une composition parfumante permettant de masquer, d'améliorer, l'odeur du DMSO, une procédure de test olfactif a été mise au point. Cette procédure permet de classer différentes formulations de manière hédonique.

### Conditions opératoires :

Pour réaliser ce test olfactif, on utilise des fûts en polyéthylène (PE) de 30 litres, chacun muni d'un couvercle dans lequel est découpée une trappe d'environ 10 cm x 10 cm, permettant à un opérateur (panéliste) de sentir les vapeurs contenues dans le fût.

Dans chacun des fûts est placé un cristallisoir contenant 2 feuilles de papier absorbant (papier chromatographique). Sur chaque feuille est versé 1 mL de composition à tester. Les fûts sont conservés fermés pendant 24 heures à température ambiante. L'évaluation est réalisée ensuite en aveugle.

Les panélistes, au nombre de 10, viennent à tour de rôle pour tester quelques produits par séance (au maximum 3 produits par séance). Ils commencent par sentir le fût dans lequel se trouve le DMSO de référence de cette étude, puis une des compositions à tester.

Les panélistes attribuent, selon leur préférence, une note à chacune des compositions à tester, par rapport à la référence qui a reçu arbitrairement la note 5. Les notes données par les panélistes vont de 1 (produit le plus agréable) à 10 (produit le plus désagréable).

### Préparation des échantillons de test :

Le DMSO de référence est un DMSO industriel de pureté égale à 99.97% produit par Arkema, puis additivé de 50 ppm de sulfure de diméthyle (DMS). Cet échantillon est nommé B₁.

Le même lot de DMSO additivé de 50 ppm de sulfure de diméthyle est additionné de 700 ppm de la composition parfumante C*ᵢ* selon l'invention. Cet échantillon est nommé B₂.

Les résultats du test olfactif sont reproduits dans le tableau 1 suivant :

**-- Tableau 1 --**

| ***Échantillon à tester*** | ***Moyenne*** | ***Écart-type*** | ***Groupe*** |
|---|---|---|---|
| B₁ | 5,93 | 1,33 | A |
| B₂ | 2,75 | 1,52 | B |

Le traitement statistique de ces résultats permet de calculer l'écart-type et de classer les échantillons en deux groupes en étudiant la PPDS (plus petite différence significative) donnée dans ce test à 1,01.

Le test PPDS est un test statistique de comparaison de moyennes et permet de déterminer si les moyennes de 2 échantillons sont significativement différentes ou non, d'un point de vue statistique.

Dans les exemples de la présente invention, le paramétrage statistique utilisé est fixé à 95%. Si les moyennes ne sont pas significativement différentes, les 2 échantillons sont classés dans un même groupe. Si les moyennes sont significativement différentes, les 2 échantillons constituent 2 groupes séparés (A et B dans les exemples illustratifs de l'invention).

La même opération est effectuée pour comparer tous les échantillons, ce qui permet *in fine* d'arriver à 1, 2 ou plusieurs groupes, chacun constitué d'échantillons dont les notes moyennes ne sont pas significativement différentes. Ces différents traitements sont réalisés à l'aide du logiciel FIZZ version 2.01 (Biosystèmes, Couternon, France).

Il y a donc une différence statistique très significative indiquant une perception de l'odeur nettement plus agréable de l'échantillon B₂ que de l'échantillon B₁.

### Exemple 2 : Composition à base de DMSO à odeur masquée

Le même DMSO industriel que celui de l'exemple 1, de pureté 99.97% produit par Arkema, est testé sans ajout des 50 ppm de DMS, suivant le test olfactif décrit à l'exemple 1. Cet échantillon est nommé C₁.

À ce même DMSO industriel est additionné 150 ppm de la composition parfumante C*ᵢ* selon l'invention. Cet échantillon est nommé C₂.

Les résultats du test olfactif sur C₁ et C₂ indiquent que l'échantillon C₂ est jugé statistiquement nettement plus agréable que l'échantillon C₁.

### Exemple 3 : Composition à base de DMSO à odeur masquée

Le même DMSO industriel que celui de l'exemple 1, de pureté 99.97% produit par Arkema, est testé sans ajout des 50 ppm de DMS, suivant le test olfactif décrit à l'exemple 1. Cet échantillon est nommé C₁.

À ce même DMSO industriel est additionné 50 ppm de la composition parfumante C*ᵢ* selon l'invention. Cet échantillon est nommé D₂.

À ce même DMSO industriel C₁ est additionné 50 ppm d'une composition parfumante constituée de 100% d'essence de pin. L'essence de pin étant un mélange de monoterpènes et de cinéols : terpinolène, dipentène, cinéol-1,4 et cinéol-1,8. Cet échantillon est nommé D₃.

Les résultats du test olfactif sont reproduits dans le tableau 2 suivant :

**-- Tableau 2 --**

| ***Échantillon à tester*** | ***Moyenne*** | ***Groupe*** |
|---|---|---|
| C₁ | 5 | A |
| D₂ | 3,68 | B |
| D₃ | 4 | B |

Une composition parfumante à base d'essence de pin permet donc de rendre l'odeur de l'échantillon C₁ plus agréable comme la composition parfumante *Ci* selon l'invention.

## Revendications

1. Composition de solvant comprenant :
a) au moins 50% et de préférence au moins 70%, de manière tout à fait préférée au moins 80% en poids d'au moins un oxyde de sulfure organique de formule générale (1) : 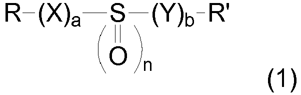 dans laquelle
- X et Y, identiques ou différents, sont choisis, indépendamment l'un de l'autre, parmi oxygène, soufre, -SO-, -SO₂-, -NH-, et -NR"- ;
- a et b, identiques ou différents, représentent, indépendamment l'un de l'autre, 0 ou 1 ; n est égal à 1 ou 2 ;
- R, R' et R", identiques ou différents, sont choisis, indépendamment les uns des autres, parmi un radical alkyle, linéaire ou ramifié, contenant de 1 à 12 atomes de carbone, un radical alcényle, linéaire ou ramifié, contenant de 2 à 12 atomes de carbone, et un radical aryle contenant de 6 à 10 atomes de carbone ; R, R' et R" pouvant éventuellement être substitués par des radicaux choisis parmi alkyle, alcényle, aryle, halogène, et pouvant éventuellement contenir un ou plusieurs hétéroatomes choisis parmi O, S, N, P et Si ; R et R' pouvant en outre former ensemble et avec les atomes qui les portent, une structure cyclique hydrocarbonée et contenant éventuellement un ou plusieurs hétéroatomes choisis parmi O, S et N, ladite structure cyclique comportant au total 5, 6, 7, 8 ou 9 sommets ; et
b) de 10 ppm à 2%, de préférence de 10 ppm à 1 % en poids par rapport au poids total de la composition, d'au moins un agent masquant d'odeur comprenant au moins un composé choisi parmi les mono-esters, les di- et/ou tri-esters, les alcools, les aldéhydes, les cétones et les terpènes.

2. Composition selon la revendication 1, dans laquelle la teneur en agent(s) masquant d'odeur b) est comprise entre 0,001% et 0,2% en poids par rapport au poids total de la composition, de préférence comprise entre 100 ppm et 1000 ppm, par exemple 500 ppm en poids.

3. Composition selon la revendication 1 ou la revendication 2, dans laquelle a représente 0, -(Y)_{b}- représente -(S)ₓ-, où x représente 0 ou 1, de préférence 0, et R et R' sont identiques et sont choisis parmi un radical alkyle linéaire ou ramifié comportant de 1 à 12 atomes de carbone, de préférence de 1 à 6 atomes de carbone, de préférence encore de 1 à 4 atomes de carbone, un radical alcényle linéaire ou ramifié comportant de 2 à 12 atomes de carbone, de préférence de 2 à 6 atomes de carbone, de préférence encore de 2 à 4 atomes de carbone, et un radical aryle, de préférence phényle.

4. Composition selon l'une quelconque des revendications précédentes, dans laquelle le composant a) de la composition selon la présente invention répond à la formule (1') : 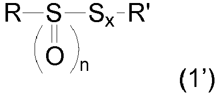 dans laquelle R est choisi parmi un radical alkyle, linéaire ou ramifié, contenant de 1 à 4 atomes de carbone, un radical alcényle, linéaire ou ramifié, contenant de 2 à 4 atomes de carbone, et un radical aryle, de préférence phényle, n est égal à 1 ou 2 ; x représente 0 ou 1 ; R' est choisi parmi un radical alkyle, linéaire ou ramifié, contenant de 1 à 4 atomes de carbone, un radical alcénylène, linéaire ou ramifié, contenant de 2 à 4 atomes de carbone, et un radical aryle, de préférence phényle.

5. Composition selon l'une quelconque des revendications précédentes, dans laquelle le composant a) de la composition selon la présente invention répond à la formule (1a) 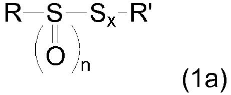 dans laquelle R et R', identiques ou différents, sont choisis parmi un radical alkyle, linéaire ou ramifié, contenant de 1 à 4 atomes de carbone, un radical alcényle, linéaire ou ramifié, contenant de 2 à 4 atomes de carbone et un radical phényle; n est égal à 1 ou 2 ; et x est égal à 0.

6. Composition selon l'une quelconque des revendications précédentes, dans laquelle le composant a) est le DMSO.

7. Composition selon l'une quelconque des revendications précédentes, dans laquelle l'agent masquant d'odeur b) comprend un ou plusieurs des composés choisis parmi :
b1) les mono-esters ;
b2) les di- et/ou tri-esters ;
b3) les alcools, avantageusement les mono-alcools, comprenant de 1 à 30 atomes de carbone, de préférence de 6 à 20 atomes de carbone, de préférence encore de 8 à 11 atomes de carbone, lesdits atomes de carbone formant une chaîne linéaire ou ramifiée comportant éventuellement une ou plusieurs insaturation(s) sous forme de double(s) liaison(s), et comportant éventuellement une structure cyclique à 5 ou 6 chaînons, saturée, ou totalement ou partiellement insaturée ;
b4) les aldéhydes et les cétones de formule R^{a}-CO-R^{b}, dans laquelle R^{a} représente une chaîne hydrocarbonée comportant de 1 à 6 atomes de carbone, linéaire ou ramifié, éventuellement comprenant une ou plusieurs insaturation(s) sous forme de double(s) liaison(s), et R^{b} représente l'atome d'hydrogène, une chaîne hydrocarbonée cyclique ou bien une chaîne hydrocarbonée linéaire ou ramifiée éventuellement, mais de préférence, substituée par une structure cyclique, R^{b} comportant de 6 à 12 atomes de carbone, comprenant éventuellement une ou plusieurs insaturation(s) sous forme de double(s) liaison(s) et étant éventuellement substitué par un ou plusieurs groupes hydroxyle ; et
b5) les terpènes.

8. Composition selon la revendication 7, dans laquelle b1) est choisi parmi les esters d'acides en C₂-C₂₀ saturés ou insaturés, tels que les acétates, propionates, butyrates, méthylbutyrates, pentanoates, hexanoates, heptanoates, caproates, oléates, linoléates, linolénates, d'éthyle, de propyle, de butyle, de pentyle, de 2-méthylbutyle, d'*i*so-amyle, d'hexyle, de benzyle, de phényléthyle, de menthyle, de carvyle, et autres, ainsi que leurs mélanges.

9. Composition selon la revendication 7, dans laquelle b2) est choisi parmi les *ortho*-phtalates, (de préférence l'*ortho*-phtalate de diéthyle), les citrates (de préférence le citrate de triéthyle) et les malonates (de préférence le malonate de diéthyle).

10. Composition selon la revendication 7, dans laquelle b3) est choisi parmi le menthol, le néo-menthol, l'alcool phényléthylique, l'alcool benzylique, le citronellol, le dihydromyrcénol, le dihydroterpinéol, le dimétol, l'éthyl-linalol, le géraniol, le linalol, le tétrahydrolinalol, le tétrahydromyrcénol, le nérol, ainsi que les mélanges de deux ou plusieurs d'entre eux.

11. Composition selon la revendication 7, dans laquelle b4) est choisi parmi le propionaldéhyde, le butyraldéhyde, le valéraldéhyde, le capraldéhyde, le benzaldéhyde, le géranial, le néral, le citronellal, et de manière générale les aldéhydes ayant des groupements hydrocarbonés comportant une ou plusieurs insaturations de type oléfinique, la menthone, l'iso-menthone, le 1,8-cinéole, l'ascaridole, la flavonone, les damascones, les damascénones, les ionones, les irisones, les méthylionones, et la frambinone, ainsi que leurs mélanges de deux ou plusieurs d'entre eux en toutes proportions.

12. Composition selon la revendication 7, dans laquelle b5) est choisi parmi les terpinènes, le myrcène, le limonène, le terpinolène, les pinènes, le sabinène, le camphène, les mélanges de deux ou plusieurs d'entre eux, ainsi que les essences à base de terpènes, notamment celles comprenant ces ingrédients.

13. Composition selon l'une quelconque des revendications précédentes, dans laquelle l'agent masquant d'odeur est choisi parmi les agents masquant d'odeurs qui comprennent :
- au moins un composant b1) ;
- au moins un composant b1) et au moins un composant b2) ;
- au moins un composant b1) et au moins un composant b3) ;
- au moins un composant b1) et au moins un composant b4) ;
- au moins un composant b1) et au moins un composant b5) ;
- au moins un composant b1), au moins un composant b2) et au moins un composant b3) ;
- au moins un composant b1), au moins un composant b2) et au moins un composant b4) ;
- au moins un composant b1), au moins un composant b2) et au moins un composant b5) ;
- au moins un composant b1), au moins un composant b3) et au moins un composant b4) ;
- au moins un composant b1), au moins un composant b3) et au moins un composant b5) ;
- au moins un composant b1), au moins un composant b2), au moins un composant b3) et au moins un composant b4) ;
- au moins un composant b1), au moins un composant b2), au moins un composant b3) et au moins un composant b5) ;
- au moins un composant b1), au moins un composant b2), au moins un composant b3), au moins un composant b4) et au moins un composant b5) ;
- au moins un composant b2) ;
- au moins un composant b2) et au moins un composant b3) ;
- au moins un composant b2) et au moins un composant b4) ;
- au moins un composant b2) et au moins un composant b5) ;
- au moins un composant b2), au moins un composant b3) et au moins un composant b4) ;
- au moins un composant b2), au moins un composant b3) et au moins un composant b5) ;
- au moins un composant b2), au moins un composant b4) et au moins un composant b5) ;
- au moins un composant b2), au moins un composant b3), au moins un composant b4) et au moins un composant b5) ;
- au moins un composant b3) ;
- au moins un composant b3) et au moins un composant b4) ;
- au moins un composant b3) et au moins un composant b5) ;
- au moins un composant b3), au moins un composant b4) et au moins un composant b5) ;
- au moins un composant b4) ;
- au moins un composant b4) et au moins un composant b5) ; et
- au moins un composant b5).

14. Composition selon l'une quelconque des revendications précédentes, dans laquelle l'agent masquant d'odeur est choisi parmi les agents masquant d'odeurs qui comprennent :
- au moins un composant b1), au moins un composant b2) et au moins un composant b3) ;
- au moins un composant b1), au moins un composant b2) et au moins un composant b4) ;
- au moins un composant b1), au moins un composant b2) et au moins un composant b5) ;
- au moins un composant b1), au moins un composant b2), au moins un composant b3) et au moins un composant b4) ;
- au moins un composant b1), au moins un composant b2), au moins un composant b3) et au moins un composant b5) ; et
- au moins un composant b1), au moins un composant b2), au moins un composant b3), au moins un composant b4) et au moins un composant b5).

15. Composition selon l'une quelconque des revendications précédentes, dans laquelle l'agent masquant d'odeur comprend de 1% à 40%, de préférence de 2% à 35%, de préférence encore de 5% à 30% en poids d'au moins un mono-ester b1), par rapport au poids total de la composition b).

16. Composition selon l'une quelconque des revendications précédentes, dans laquelle l'agent masquant d'odeur comprend de 10% à 70% en poids, de préférence de 15% à 65% en poids, de préférence encore de 20% à 60% en poids d'au moins un di- et/ou tri-ester b2), par rapport au poids total de la composition b).

17. Composition selon l'une quelconque des revendications précédentes, dans laquelle l'agent masquant d'odeur comprend de 1% à 30%, de préférence de 5% à 25% en poids, d'au moins un alcool b3), par rapport au poids total de la composition b).

18. Composition selon l'une quelconque des revendications précédentes, dans laquelle l'agent masquant d'odeur comprend de 0,5% à 20%, de préférence de 1% à 10% en poids, d'au moins un aldéhyde et/ou une cétone b4), par rapport au poids total de la composition b).

19. Composition selon l'une quelconque des revendications précédentes, dans laquelle l'agent masquant d'odeur comprend jusqu'à 20%, de préférence de 1% à 10% en poids, d'au moins un terpène indiqué sous b5), par rapport au poids total de la composition b).

20. Composition selon l'une quelconque des revendications précédentes, dans laquelle l'agent masquant d'odeur comprend :
b1) de 1 % à 40% en poids d'au moins un mono-ester ;
b2) de 10% à 70% en poids d'au moins un di- et/ou tri-ester ;
b3) de 1% à 30% en poids d'au moins un alcool ;
b4) de 0,5% à 20% en poids d'au moins un aldéhyde et/ou une cétone de formule R^{a}-CO-R^{b}, dans laquelle R^{a} représente une chaîne hydrocarbonée comportant de 1 à 6 atomes de carbone, linéaire ou ramifié, éventuellement comprenant une ou plusieurs insaturation(s) sous forme de double(s) liaison(s), et R^{b} représente une chaîne hydrocarbonée cyclique ou bien une chaîne hydrocarbonée linéaire ou ramifiée éventuellement mais de préférence substituée par une structure cyclique, R^{b} comportant de 6 à 12 atomes de carbone, comprenant éventuellement une ou plusieurs insaturation(s) sous forme de double(s) liaison(s) et étant éventuellement substitué par un ou plusieurs groupes hydroxyle ; et
b5) éventuellement jusqu'à 20% en poids d'au moins un terpène.

21. Composition selon l'une quelconque des revendications précédentes, dans laquelle l'agent masquant d'odeur comprend :
- de 5% à 30% en poids d'au moins un mono-ester b1), choisi parmi l'acétate d'*i*so-amyle, le méthyl-2-butyrate éthyle, le butyrate d'*i*so-amyle, l'acétate de phényléthyle, le caproate d'éthyle, l'acétate de benzyle, l'acétate d'hexyle et leurs mélanges ;
- de 20% à 60% en poids d'au moins un di- et/ou tri-ester b2) choisi(s) parmi les *ortho*-phtalates, tel que l'*ortho*-phtalate de diéthyle ; les citrates, tels que le citrate de triéthyle ; et les malonates, tel que le malonate de diéthyle, et leurs mélanges ;
- de 5% à 25% en poids d'au moins un alcool, de préférence d'au moins deux alcools, de préférence encore d'au moins trois alcools, tels que décrits précédemment sous b3) ;
- de 1 % à 10% en poids d'au moins une cétone, de préférence au moins deux cétones, de préférence encore au moins trois cétones, telles que décrites précédemment sous b4) ; et
- de 1% à 10% en poids d'au moins un, de préférence au moins deux, de préférence un mélange de, terpènes référencés plus haut sous b5).

22. Composition selon l'une quelconque des revendications précédentes, comprenant :
a) de 50% à 95%, de préférence de 70% à 90%, de manière tout à fait préférée 80% en poids d'au moins un oxyde de sulfure organique de formule générale (1) ;
b) de 10 ppm, à 2%, de préférence de 10 ppm à 1% en poids par rapport au poids total de la composition, d'au moins un agent masquant d'odeur b) ; et
c) de 5% à 50%, de préférence de 10% à 30%, de manière tout à fait préférée 20% en poids d'au moins un co-solvant.

23. Composition selon la revendication 22, dans laquelle le co-solvant est choisi parmi les composés solubles au moins partiellement ou totalement dans les oxydes de sulfures organiques a), de préférence choisi parmi les carbonates, esters, cétones, amines, amides, et les alcools, de préférence encore parmi les alcools, de préférence saturés, de préférence comportant de 1 à 10 atomes de carbone, et de préférence encore parmi le méthanol, l'éthanol, le propanol, le butanol, le pentanol, l'éthylène-glycol, le propylène-glycol et le glycérol (propane-1,2,3-triol).

24. Composition selon la revendication 22, comprenant :
a) 80% en poids de DMSO ;
b) de 10 ppm, à 2%, de préférence de 10 ppm à 1% en poids par rapport au poids total de la composition, de l'agent masquant comprenant :
- de 5% à 30% en poids d'au moins un mono-ester b1), choisi parmi l'acétate d'*i*so-amyle, le méthyl-2-butyrate éthyle, le butyrate d'*i*so-amyle, l'acétate de phényléthyle, le caproate d'éthyle, l'acétate de benzyle, l'acétate d'hexyle et leurs mélanges ;
- de 20% à 60% en poids d'au moins un di- et/ou tri-ester b2) choisi(s) parmi les *ortho*-phtalates, tel que l'*ortho*-phtalate de diéthyle ; les citrates, tels que le citrate de triéthyle ; et les malonates, tel que le malonate de diéthyle, et leurs mélanges ;
- de 5% à 25% en poids d'au moins un alcool, de préférence d'au moins deux alcools, de préférence encore d'au moins trois alcools, tels que décrits précédemment sous b3) ;
- de 1% à 10% en poids d'au moins une cétone, de préférence au moins deux cétones, de préférence encore au moins trois cétones, telles que décrites précédemment sous b4) ; et
- de 1% à 10% en poids d'au moins un, de préférence au moins deux, de préférence un mélange de, terpènes référencés plus haut sous b5) ; et
c) 20% en poids de glycérol.

## Patentansprüche

1. Lösungsmittelzusammensetzung, umfassend:
a) mindestens 50 Gew.-% und vorzugsweise mindestens 70 Gew.-%, ganz besonders bevorzugt mindestens 80 Gew.-% mindestens eines organischen Schwefeloxids der allgemeinen Formel (1): 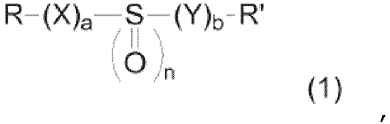 worin
- X und Y, die gleich oder verschieden sind, unabhängig voneinander aus Sauerstoff, Schwefel, -SO-, -SO₂-, -NH- und NR''- ausgewählt sind;
- a und b, die gleich oder verschieden sind, unabhängig voneinander für 0 oder 1 stehen; n gleich 1 oder 2 ist;
- R, R' und R'', die gleich oder verschieden sind, unabhängig voneinander aus einem geraden oder verzweigten Alkylrest mit 1 bis 12 Kohlenstoffatomen, einem geraden oder verzweigten Alkenylrest mit 2 bis 12 Kohlenstoffatomen und einem Arylrest mit 6 bis 10 Kohlenstoffatomen ausgewählt sind; wobei R, R' und R'' gegebenenfalls mit Resten substituiert sein können, die aus Alkyl, Alkenyl, Aryl, Halogen ausgewählt sind, und gegebenenfalls ein oder mehrere Heteroatome enthalten können, die aus O, S, N, P und Si ausgewählt sind; R und R' außerdem zusammen mit den Atomen, die sie tragen, eine zyklische Kohlenwasserstoffstruktur bilden können, die gegebenenfalls ein oder mehrere Heteroatome enthält, die aus O, S und N ausgewählt sind, wobei die zyklische Struktur insgesamt 5, 6, 7, 8 oder 9 Ringglieder enthält; und
b) 10 ppm bis 2 Gew.-%, vorzugsweise 10 ppm bis 1 Gew.-%, bezogen auf das Gesamtgewicht der Zusammensetzung, von mindestens einem Geruchsmaskierungsmittel, das mindestens eine aus Monoestern, Di- und/oder Triestern, Alkoholen, Aldehyden, Ketonen und Terpenen ausgewählte Verbindung umfasst.

2. Zusammensetzung nach Anspruch 1, worin der Gehalt an Geruchsmaskierungsmittel(n) b) zwischen 0,001 Gew.-% und 0,2 Gew.-%, bezogen auf das Gesamtgewicht der Zusammensetzung, vorzugsweise zwischen 100 ppm und 1000 ppm, zum Beispiel 500 ppm, bezogen auf Gewicht, beträgt.

3. Zusammensetzung nach Anspruch 1 oder Anspruch 2, worin a für 0 steht, - (Y)_{b}- für -(S)ₓ- steht, wobei x für 0 oder 1, vorzugsweise 0, steht, und R und R' gleich sind und aus einem geraden oder verzweigten Alkylrest mit 1 bis 12 Kohlenstoffatomen, vorzugsweise von 1 bis 6 Kohlenstoffatomen, ebenfalls bevorzugt von 1 bis 4 Kohlenstoffatomen, einem geraden oder verzweigten Alkenylrest mit 2 bis 12 Kohlenstoffatomen, vorzugsweise von 2 bis 6 Kohlenstoffatomen, ebenfalls bevorzugt von 2 bis 4 Kohlenstoffatomen, und einem Aryl-, vorzugsweise Phenylrest ausgewählt sind.

4. Zusammensetzung nach einem der vorhergehenden Ansprüche, worin die Komponente a) der Zusammensetzung gemäß der vorliegenden Erfindung der folgenden Formel (1') entspricht: 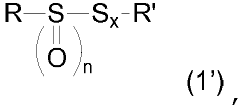 worin R aus einem geraden oder verzweigten Alkylrest mit 1 bis 4 Kohlenstoffatomen, einem geraden oder verzweigten Alkenylrest mit 2 bis 4 Kohlenstoffatomen und einem Aryl-, vorzugsweise Phenylrest ausgewählt ist, n gleich 1 oder 2 ist; x für 0 oder 1 steht; R' aus einem geraden oder verzweigten Alkylrest mit 1 bis 4 Kohlenstoffatomen, einem geraden oder verzweigten Alkenylenrest mit 2 bis 4 Kohlenstoffatomen und einem Aryl-, vorzugsweise Phenylrest ausgewählt ist.

5. Zusammensetzung nach einem der vorhergehenden Ansprüche, worin die Komponente a) der Zusammensetzung gemäß der vorliegenden Erfindung der Formel (1a) entspricht 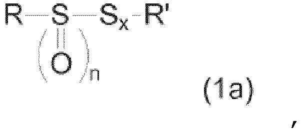 worin R und R', die gleich oder verschieden sind, aus einem geraden oder verzweigten Alkylrest mit 1 bis 4 Kohlenstoffatomen, einem geraden oder verzweigten Alkenylrest mit 2 bis 4 Kohlenstoffatomen und einem Phenylrest ausgewählt sind; n gleich 1 oder 2 ist und x gleich 0 ist.

6. Zusammensetzung nach einem der vorhergehenden Ansprüche, worin die Komponente a) DMSO ist.

7. Zusammensetzung nach einem der vorhergehenden Ansprüche, worin das Geruchsmaskierungsmittel b) eine oder mehrere Verbindungen umfasst, ausgewählt aus:
b1) Monoestern;
b2) Di- und/oder Triestern;
b3) Alkoholen, vorzugsweise Monoalkoholen, umfassend von 1 bis 30 Kohlenstoffatomen, vorzugsweise von 6 bis 20 Kohlenstoffatomen, ebenfalls bevorzugt von 8 bis 11 Kohlenstoffatomen, wobei die Kohlenstoffatome eine gerade oder verzweigte Kette bilden, die gegebenenfalls eine oder mehrere Ungesättigtheit(en) in Form von (einer) Doppelbindung(en) trägt und gegebenenfalls eine gesättigte oder vollständig oder partiell ungesättigte zyklische Struktur mit 5 oder 6 Ringgliedern trägt;
b4) Aldehyden und Ketonen der Formel R^{a}-CO-R^{b}, worin R^{a} für eine gerade oder verzweigte Kohlenwasserstoffkette mit 1 bis 6 Kohlenstoffatomen steht, die gegebenenfalls eine oder mehrere Ungesättigtheit(en) in Form von (einer) Doppelbindung(en) trägt, und R^{b} für ein Wasserstoffatom, eine zyklische Kohlenwasserstoffkette oder auch eine gerade oder verzweigte Kohlenwasserstoffkette steht, die gegebenenfalls, jedoch bevorzugt, mit einer zyklischen Struktur substituiert ist, wobei der Rest R^{b}, der 6 bis 12 Kohlenstoffatome enthält, gegebenenfalls eine oder mehrere Ungesättigtheit(en) in Form von (einer) Doppelbindung(en) trägt und gegebenenfalls mit einer oder mehreren Hydroxylgruppen substituiert ist; und
b5) Terpenen.

8. Zusammensetzung nach Anspruch 7, worin b1) aus Estern gesättigter oder ungesättigter C2-C20-Säuren, wie Ethyl-, Propyl-, Butyl-, Pentyl-, 2-Methylbutyl-, iso-Amyl-, Hexyl-, Benzyl-, Phenylethyl-, Menthyl-, Carvylacetate, -propionate, -butyrate, -methylbutyrate, -pentanoate, -hexanoate, -heptanoate, -caproate, - oleate, -linoleate, -linolenate und andere sowie deren Gemische, ausgewählt ist.

9. Zusammensetzung nach Anspruch 7, worin b2) aus ortho-Phthalaten (vorzugsweise Diethyl-ortho-phthalat), Citraten (vorzugsweise Triethylcitrat) und Malonaten (vorzugsweise Diethylmalonat) ausgewählt ist.

10. Zusammensetzung nach Anspruch 7, worin b3) aus Menthol, neo-Menthol, Phenylthylalkohol, Benzylalkohol, Citronellol, Dihydromyrcenol, Dihydroterpineol, Dimetol, Ethyllinalol, Geraniol, Linalol, Tetrahydrolinalol, Tetrahydromyrcenol, Nerol sowie Gemischen von zwei oder mehreren von diesen ausgewählt ist.

11. Zusammensetzung nach Anspruch 7, worin b4) aus Propionaldehyd, Butyraldehyd, Valeraldehyd, Capraldehyd, Benzaldehyd, Geranial, Neral, Citronellal und allgemein Aldehyden mit Kohlenwasserstoffgruppen, die eine oder mehrere Ungesättigtheiten des olefinischen Typs tragen, Menthon, iso-Menthon, 1,8-Cineol, Ascaridol, Flavonon, Damasconen, Damascenonen, Iononen, Irisonen, Methyliononen und Frambinon sowie deren Gemischen von zwei oder mehreren von diesen in allen Verhältnissen ausgewählt ist.

12. Zusammensetzung nach Anspruch 7, worin b5) aus Terpinenen, Myrcen, Limonen, Terpinolen, Pinenen, Sabinen, Camphen, Gemischen von zwei oder mehreren von diesen sowie Essenzen auf der Basis von Terpenen, insbesondere denjenigen, die diese Inhaltsstoffe umfassen, ausgewählt ist.

13. Zusammensetzung nach einem der vorhergehenden Ansprüche, worin das Geruchsmaskierungsmittel aus Geruchsmaskierungsmitteln ausgewählt ist, die Folgendes umfassen:
- mindestens eine Komponente b1);
- mindestens eine Komponente b1) und mindestens eine Komponente b2);
- mindestens eine Komponente b1) und mindestens eine Komponente b3);
- mindestens eine Komponente b1) und mindestens eine Komponente b4);
- mindestens eine Komponente b1) und mindestens eine Komponente b5);
- mindestens eine Komponente b1), mindestens eine Komponente b2) und mindestens eine Komponente b3);
- mindestens eine Komponente b1), mindestens eine Komponente b2) und mindestens eine Komponente b4);
- mindestens eine Komponente b1), mindestens eine Komponente b2) und mindestens eine Komponente b5);
- mindestens eine Komponente b1), mindestens eine Komponente b3) und mindestens eine Komponente b4);
- mindestens eine Komponente b1), mindestens eine Komponente b3) und mindestens eine Komponente b5);
- mindestens eine Komponente b1), mindestens eine Komponente b2), mindestens eine Komponente b3) und mindestens eine Komponente b4);
- mindestens eine Komponente b1), mindestens eine Komponente b2), mindestens eine Komponente b3) und mindestens eine Komponente b5);
- mindestens eine Komponente b1), mindestens eine Komponente b2), mindestens eine Komponente b3), mindestens eine Komponente b4) und mindestens eine Komponente b5);
- mindestens eine Komponente b2);
- mindestens eine Komponente b2) und mindestens eine Komponente b3);
- mindestens eine Komponente b2) und mindestens eine Komponente b4);
- mindestens eine Komponente b2) und mindestens eine Komponente b5);
- mindestens eine Komponente b2), mindestens eine Komponente b3) und mindestens eine Komponente b4);
- mindestens eine Komponente b2), mindestens eine Komponente b3) und mindestens eine Komponente b5);
- mindestens eine Komponente b2), mindestens eine Komponente b4) und mindestens eine Komponente b5);
- mindestens eine Komponente b2), mindestens eine Komponente b3), mindestens eine Komponente b4) und mindestens eine Komponente b5);
- mindestens eine Komponente b3);
- mindestens eine Komponente b3) und mindestens eine Komponente b4);
- mindestens eine Komponente b3) und mindestens eine Komponente b5);
- mindestens eine Komponente b3), mindestens eine Komponente b4) und mindestens eine Komponente b5);
- mindestens eine Komponente b4);
- mindestens eine Komponente b4) und mindestens eine Komponente b5); und
- mindestens eine Komponente b5).

14. Zusammensetzung nach einem der vorhergehenden Ansprüche, worin das Geruchsmaskierungsmittel aus Geruchsmaskierungsmitteln ausgewählt ist, die Folgendes umfassen:
- mindestens eine Komponente b1), mindestens eine Komponente b2) und mindestens eine Komponente b3);
- mindestens eine Komponente b1), mindestens eine Komponente b2) und mindestens eine Komponente b4);
- mindestens eine Komponente b1), mindestens eine Komponente b2) und mindestens eine Komponente b5);
- mindestens eine Komponente b1), mindestens eine Komponente b2), mindestens eine Komponente b3) und mindestens eine Komponente b4);
- mindestens eine Komponente b1), mindestens eine Komponente b2), mindestens eine Komponente b3) und mindestens eine Komponente b5); und
- mindestens eine Komponente b1), mindestens eine Komponente b2), mindestens eine Komponente b3), mindestens eine Komponente b4) und mindestens eine Komponente b5).

15. Zusammensetzung nach einem der vorhergehenden Ansprüche, worin das Geruchsmaskierungsmittel 1 Gew.-% bis 40 Gew.-%, vorzugsweise 2 Gew.-% bis 35 Gew.-%, ebenfalls bevorzugt von 5 Gew.-% bis 30 Gew.-% von mindestens einem Monoester b1), bezogen auf das Gesamtgewicht der Zusammensetzung b), umfasst.

16. Zusammensetzung nach einem der vorhergehenden Ansprüche, worin das Geruchsmaskierungsmittel 10 Gew.-% bis 70 Gew.-%, vorzugsweise 15 Gew.-% bis 65 Gew.-%, ebenfalls bevorzugt von 20 Gew.-% bis 60 Gew.-% von mindestens einem Di- und/oder Triester b2), bezogen auf das Gesamtgewicht der Zusammensetzung b), umfasst.

17. Zusammensetzung nach einem der vorhergehenden Ansprüche, worin das Geruchsmaskierungsmittel 1 Gew.-% bis 30 Gew.-%, vorzugsweise 5 Gew.-% bis 25 Gew.-% von mindestens einem Alkohol b3), bezogen auf das Gesamtgewicht der Zusammensetzung b), umfasst.

18. Zusammensetzung nach einem der vorhergehenden Ansprüche, worin das Geruchsmaskierungsmittel 0,5 Gew.-% bis 20 Gew.-%, vorzugsweise 1 Gew.-% bis 10 Gew.-% von mindestens einem Aldehyd und/oder einem Keton b4), bezogen auf das Gesamtgewicht der Zusammensetzung b), umfasst.

19. Zusammensetzung nach einem der vorhergehenden Ansprüche, worin das Geruchsmaskierungsmittel bis zu 20 Gew.-%, vorzugsweise von 1 Gew.-% bis 10 Gew.-%, von mindestens einem unter b5) angegebenen Terpen, bezogen auf das Gesamtgewicht der Zusammensetzung b), umfasst.

20. Zusammensetzung nach einem der vorhergehenden Ansprüche, worin das Geruchsmaskierungsmittel Folgendes umfasst:
b1) 1 Gew.-% bis 40 Gew.-% von mindestens einem Monoester;
b2) 10 Gew.-% bis 70 Gew.-% von mindestens einem Di- und/oder Triester;
b3) 1 Gew.-% bis 30 Gew.-% von mindestens einem Alkohol;
b4) 0,5 Gew.-% bis 20 Gew.-% von mindestens einem Aldehyd und/oder einem Keton der Formel R^{a}-CO-R^{b}, worin R^{a} für eine gerade oder verzweigte Kohlenwasserstoffkette mit 1 bis 6 Kohlenstoffatomen steht, die gegebenenfalls eine oder mehrere Ungesättigtheit(en) in Form von (einer) Doppelbindung(en) umfasst, und R^{b} für eine zyklische Kohlenwasserstoffkette oder auch ein gerade oder verzweigte eine Kohlenwasserstoffkette steht, die gegebenenfalls, jedoch vorzugsweise, mit einer zyklischen Struktur substituiert ist, wobei R^{b} 6 bis 12 Kohlenstoffatome trägt, gegebenenfalls eine oder mehrere Ungesättigtheit(en) in Form von (einer) Doppelbindung(en) umfasst und gegebenenfalls mit einer oder mehreren Hydroxylgruppen substituiert ist; und
b5) gegebenenfalls bis zu 20 Gew.-% von mindestens einem Terpen.

21. Zusammensetzung nach einem der vorhergehenden Ansprüche, worin das Geruchsmaskierungsmittel Folgendes umfasst:
- 5 Gew.-% bis 30 Gew.-% von mindestens einem Monoester b1), ausgewählt aus iso-Amylacetat, Ethylmethyl-2-butyrat, iso-Amylbutyrat, Phenylethylacetat, Ethylcaproat, Benzylacetat, Hexylacetat und deren Gemischen;
- 20 Gew.-% bis 60 Gew.-% von mindestens einem Di- und/oder Triester b2), ausgewählt aus ortho-Phthalaten, wie Diethyl-ortho-phthalat; Citraten, wie Triethylcitrat; und Malonaten, wie Diethylmalonat, und deren Gemischen;
- 5 Gew.-% bis 25 Gew.-% von mindestens einem Alkohol, vorzugsweise mindestens zwei Alkoholen, ebenfalls bevorzugt mindestens drei Alkoholen, wie vorstehend unter b3) beschrieben;
- 1 Gew.-% bis 10 Gew.-% von mindestens einem Keton, vorzugsweise mindestens zwei Ketonen, ebenfalls bevorzugt mindestens drei Ketonen, wie vorstehend unter b4) beschrieben; und
- 1 Gew.-% bis 10 Gew.-% von mindestens einem, vorzugsweise mindestens zwei, vorzugsweise einem Gemisch von Terpenen, die vorstehend unter b5) angegeben sind.

22. Zusammensetzung nach einem der vorhergehenden Ansprüche, umfassend:
a) 50 Gew.-% bis 95 Gew.-%, vorzugsweise 70 Gew.-% bis 90 Gew.-%, ganz besonders bevorzugt 80 Gew.-% von mindestens einem organischen Schwefeloxid der allgemeinen Formel (1);
b) 10 ppm bis 2 Gew.-%, vorzugsweise 10 ppm bis 1 Gew.-%, bezogen auf das Gesamtgewicht der Zusammensetzung, von mindestens einem Geruchsmaskierungsmittel b); und
c) 5 Gew.-% bis 50 Gew.-%, vorzugsweise 10 Gew.-% bis 30 Gew.-%, ganz besonders bevorzugt 20 Gew.-% von mindestens einem Co-Lösungsmittel.

23. Zusammensetzung nach Anspruch 22, worin das Co-Lösungsmittel aus Verbindungen ausgewählt ist, die zumindest partiell oder vollständig in organischen Schwefeloxiden a) löslich sind, vorzugsweise ausgewählt aus Carbonaten, Estern, Ketonen, Aminen, Amiden und Alkoholen, ebenfalls bevorzugt aus, vorzugsweise gesättigten, Alkoholen, die vorzugsweise 1 bis 10 Kohlenstoffatome tragen, und ebenfalls bevorzugt aus Methanol, Ethanol, Propanol, Butanol, Pentanol, Ethylenglykol, Propylenglykol und Glycerin (Propan-1,2,3-triol).

24. Zusammensetzung nach Anspruch 22, umfassend:
a) 80 Gew.-% DMSO;
b) 10 ppm bis 2 Gew.-%, vorzugsweise 10 ppm bis 1 Gew.-%, bezogen auf das Gesamtgewicht der Zusammensetzung, des Maskierungsmittels, das Folgendes umfasst:
- 5 Gew.-% bis 30 Gew.-% von mindestens einem Monoester b1), ausgewählt aus iso-Amylacetat, Ethylmethyl-2-butyrat, iso-Amylbutyrat, Phenylethylacetat, Ethylcaproat, Benzylacetat, Hexylacetat und deren Gemischen;
- 20 Gew.-% bis 60 Gew.-% von mindestens einem Di- und/oder Triester b2), ausgewählt aus ortho-Phthalaten, wie Diethyl-ortho-phthalat; Citraten, wie Triethylcitrat; und Malonaten, wie Diethylmalonat, und deren Gemischen;
- 5 Gew.-% bis 25 Gew.-% von mindestens einem Alkohol, vorzugsweise mindestens zwei Alkoholen, ebenfalls bevorzugt mindestens drei Alkoholen, wie vorstehend unter b3) beschrieben;
- 1 Gew.-% bis 10 Gew.-% von mindestens einem Keton, vorzugsweise mindestens zwei Ketonen, ebenfalls bevorzugt mindestens drei Ketonen, wie vorstehend unter b4) beschrieben; und
- 1 Gew.-% bis 10 Gew.-% von mindestens einem, vorzugsweise mindestens zwei, vorzugsweise einem Gemisch von Terpenen, die vorstehend unter b5) angegeben sind; und
c) 20 Gew.-% Glycerin.

## Claims

1. Solvent composition comprising:
a) at least 50% and preferably at least 70%, and most preferably at least 80% by weight of at least one oxide of an organic sulfide of general formula (1): 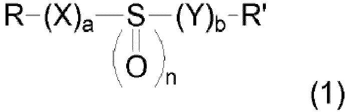 in which
- X and Y, which may be identical or different, are chosen, independently of each other, from oxygen, sulfur, -SO-, -SO₂-, -NH- and -NR"-;
- a and b, which may be identical or different, represent, independently of each other, 0 or 1; n is equal to 1 or 2;
- R, R' and R", which may be identical or different, are chosen, independently of each other, from a linear or branched alkyl radical containing from 1 to 12 carbon atoms, a linear or branched alkenyl radical containing from 2 to 12 carbon atoms, and an aryl radical containing from 6 to 10 carbon atoms; R, R' and R" possibly being substituted with radicals chosen from alkyl, alkenyl, aryl and halogen, and possibly containing one or more heteroatoms chosen from O, S, N, P and Si; R and R' also possibly forming, together with the atoms that bear them, a hydrocarbon-based cyclic structure optionally containing one or more heteroatoms chosen from O, S and N, said cyclic structure comprising in total 5, 6, 7, 8 or 9 ring members; and
b) from 10 ppm to 2%, preferably from 10 ppm to 1%, by weight relative to the total weight of the composition, of at least one odor-masking agent comprising at least one compound chosen from monoesters, diesters and/or triesters, alcohols, aldehydes, ketones and terpenes.

2. Composition according to Claim 1, in which the content of odor-masking agent(s) b) is between 0.001% and 0.2% by weight relative to the total weight of the composition and preferably between 100 ppm and 1000 ppm, for example 500 ppm by weight.

3. Composition according to Claim 1 or 2, in which a represents 0, -(Y)_{b}- represents -(S)ₓ-, in which x represents 0 or 1, preferably 0, and R and R' are identical and chosen from a linear or branched alkyl radical comprising from 1 to 12 carbon atoms, preferably from 1 to 6 carbon atoms and more preferably from 1 to 4 carbon atoms, a linear or branched alkenyl radical comprising from 2 to 12 carbon atoms, preferably from 2 to 6 carbon atoms and more preferably from 2 to 4 carbon atoms, and an aryl radical, preferably phenyl.

4. Composition according to any one of the preceding claims, in which component a) of the composition according to the present invention corresponds to formula (1') : 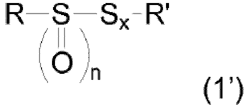 in which R is chosen from a linear or branched alkyl radical containing from 1 to 4 carbon atoms, a linear or branched alkenyl radical containing from 2 to 4 carbon atoms, and an aryl radical, preferably phenyl, n is equal to 1 or 2; x represents 0 or 1; R' is chosen from a linear or branched alkyl radical containing from 1 to 4 carbon atoms, a linear or branched alkenylene radical containing from 2 to 4 carbon atoms, and an aryl radical, preferably phenyl.

5. Composition according to any one of the preceding claims, in which component a) of the composition according to the present invention corresponds to formula (1a) 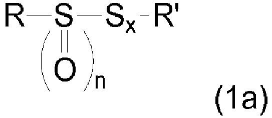 in which R and R', which may be identical or different, are chosen from a linear or branched alkyl radical containing from 1 to 4 carbon atoms, a linear or branched alkenyl radical containing from 2 to 4 carbon atoms, and a phenyl radical; n is equal to 1 or 2; and x is equal to 0.

6. Composition according to any one of the preceding claims, in which component a) is DMSO.

7. Composition according to any one of the preceding claims, in which the odor-masking agent b) comprises one or more compounds chosen from:
b1) monoesters;
b2) diesters and/or triesters;
b3) alcohols, advantageously monoalcohols, comprising from 1 to 30 carbon atoms, preferably from 6 to 20 carbon atoms and more preferably from 8 to 11 carbon atoms, said carbon atoms forming a linear or branched chain optionally comprising one or more unsaturation(s) in the form of double bonds, and optionally comprising a 5- or 6-membered cyclic structure, which is saturated or partially or totally unsaturated;
b4) aldehydes and ketones of formula R^{a}-CO-R^{b}, in which R^{a} represents a linear or branched hydrocarbon-based chain comprising from 1 to 6 carbon atoms, optionally comprising one or more unsaturation(s) in the form of double bonds, and R^{b} represents a hydrogen atom, a cyclic hydrocarbon-based chain or a linear or branched hydrocarbon-based chain optionally, but preferably, substituted with a cyclic structure, R^{b} comprising from 6 to 12 carbon atoms, optionally comprising one or more unsaturation(s) in the form of double bonds and being optionally substituted with one or more hydroxyl groups; and
b5) terpenes.

8. Composition according to Claim 7, in which b1) is chosen from esters of saturated or unsaturated C₂-C₂₀ acids, such as acetates, propionates, butyrates, methylbutyrates, pentanoates, hexanoates, heptanoates, caproates, oleates, linoleates or linolenates of ethyl, propyl, butyl, pentyl, 2-methylbutyl, isoamyl, hexyl, benzyl, phenylethyl, menthyl, carvyl, and the like, and also mixtures thereof.

9. Composition according to Claim 7, in which b2) is chosen from ortho-phthalates (preferably diethyl *ortho-*phthalate), citrates (preferably triethyl citrate) and malonates (preferably diethyl malonate).

10. Composition according to Claim 7, in which b3) is chosen from menthol, neomenthol, phenylethyl alcohol, benzyl alcohol, citronellol, dihydromyrcenol, dihydroterpineol, dimetol, ethyllinalool, geraniol, linalool, tetrahydrolinalool, tetrahydromyrcenol, nerol, and also mixtures of two or more thereof.

11. Composition according to Claim 7, in which b4) is chosen from propionaldehyde, butyraldehyde, valeraldehyde, capraldehyde, benzaldehyde, geranial, neral, citronellal and, in general, aldehydes containing hydrocarbon-based groups comprising one or more unsaturations of olefinic type, menthone, isomenthone, 1,8-cineole, ascaridole, flavonone, damascones, damascenones, ionones, irisones, methyl-ionones, frambinone, and also mixtures of two or more thereof in all proportions.

12. Composition according to Claim 7, in which b5) is chosen from terpinenes, myrcene, limonene, terpinolene, pinenes, sabinene, camphene, mixtures of two or more thereof, and also terpene-based essences, especially those comprising these ingredients.

13. Composition according to any one of the preceding claims, in which the odor-masking agent is chosen from odor-masking agents comprising:
- at least one component b1);
- at least one component b1) and at least one component b2);
- at least one component b1) and at least one component b3) ;
- at least one component b1) and at least one component b4);
- at least one component b1) and at least one component b5) ;
- at least one component b1), at least one component b2) and at least one component b3);
- at least one component b1), at least one component b2) and at least one component b4);
- at least one component b1), at least one component b2) and at least one component b5);
- at least one component b1), at least one component b3) and at least one component b4);
- at least one component b1), at least one component b3) and at least one component b5);
- at least one component b1), at least one component b2), at least one component b3) and at least one component b4);
- at least one component b1), at least one component b2), at least one component b3) and at least one component b5);
- at least one component b1), at least one component b2), at least one component b3), at least one component b4) and at least one component b5);
- at least one component b2);
- at least one component b2) and at least one component b3) ;
- at least one component b2) and at least one component b4) ;
- at least one component b2) and at least one component b5) ;
- at least one component b2), at least one component b3) and at least one component b4);
- at least one component b2), at least one component b3) and at least one component b5);
- at least one component b2), at least one component b4) and at least one component b5);
- at least one component b2), at least one component b3), at least one component b4) and at least one component b5);
- at least one component b3);
- at least one component b3) and at least one component b4);
- at least one component b3) and at least one component b5) ;
- at least one component b3), at least one component b4) and at least one component b5);
- at least one component b4);
- at least one component b4) and at least one component b5); and
- at least one component b5).

14. Composition according to any one of the preceding claims, in which the odor-masking agent is chosen from odor-masking agents comprising:
- at least one component b1), at least one component b2) and at least one component b3);
- at least one component b1), at least one component b2) and at least one component b4);
- at least one component b1), at least one component b2) and at least one component b5);
- at least one component b1), at least one component b2), at least one component b3) and at least one component b4);
- at least one component b1), at least one component b2), at least one component b3) and at least one component b5); and
- at least one component b1), at least one component b2), at least one component b3), at least one component b4) and at least one component b5).

15. Composition according to any one of the preceding claims, in which the odor-masking agent comprises from 1% to 40%, preferably from 2% to 35% and more preferably from 5% to 30% by weight of at least one monoester b1) relative to the total weight of composition b).

16. Composition according to any one of the preceding claims, in which the odor-masking agent comprises from 10% to 70%, preferably from 15% to 65% and more preferably from 20% to 60% by weight of at least one diester and/or triester b2) relative to the total weight of composition b).

17. Composition according to any one of the preceding claims, in which the odor-masking agent comprises from 1% to 30% and preferably from 5% to 25% by weight of at least one alcohol b3) relative to the total weight of composition b).

18. Composition according to any one of the preceding claims, in which the odor-masking agent comprises from 0.5% to 20% and preferably from 1% to 10% by weight of at least one aldehyde and/or ketone b4) relative to the total weight of composition b).

19. Composition according to any one of the preceding claims, in which the odor-masking agent comprises up to 20% and preferably from 1% to 10% by weight of at least one terpene indicated in b5) relative to the total weight of composition b).

20. Composition according to any one of the preceding claims, in which the odor-masking agent comprises:
b1) from 1% to 40% by weight of at least one monoester;
b2) from 10% to 70% by weight of at least one diester and/or triester;
b3) from 1% to 30% by weight of at least one alcohol;
b4) from 0.5% to 20% by weight of at least one aldehyde and/or ketone of formula R^{a}-CO-R^{b}, in which R^{a} represents a linear or branched hydrocarbon-based chain comprising from 1 to 6 carbon atoms, optionally comprising one or more unsaturation(s) in the form of double bonds, and R^{b} represents a cyclic hydrocarbon-based chain or a linear or branched hydrocarbon-based chain optionally, but preferably, substituted with a cyclic structure, R^{b} comprising from 6 to 12 carbon atoms, optionally comprising one or more unsaturation(s) in the form of double bonds and being optionally substituted with one or more hydroxyl groups; and
b5) optionally up to 20% by weight of at least one terpene.

21. Composition according to any one of the preceding claims, in which the odor-masking agent comprises:
- from 5% to 30% by weight of at least one monoester b1), chosen from isoamyl acetate, ethyl 2-methylbutyrate, isoamyl butyrate, phenylethyl acetate, ethyl caproate, benzyl acetate and hexyl acetate, and mixtures thereof;
- from 20% to 60% by weight of at least one diester and/or triester b2) chosen from ortho-phthalates, such as diethyl ortho-phthalate; citrates, such as triethyl citrate; malonates, such as diethyl malonate, and mixtures thereof;
- from 5% to 25% by weight of at least one alcohol, preferably of at least two alcohols, and more preferably of at least three alcohols, as described previously in b3);
- from 1% to 10% by weight of at least one ketone, preferably at least two ketones, more preferably at least three ketones, as described previously in b4); and
- from 1% to 10% by weight of at least one, preferably at least two, and preferably a mixture of terpenes referenced above in b5).

22. Composition according to any one of the preceding claims, comprising:
a) from 50% to 95%, preferably from 70% to 90% and most preferably 80% by weight of at least one oxide of an organic sulfide of general formula (1);
b) from 10 ppm to 2%, preferably from 10 ppm to 1% by weight, relative to the total weight of the composition, of at least one odor-masking agent b); and
c) from 5% to 50%, preferably from 10% to 30% and most preferably 20% by weight of at least one cosolvent.

23. Composition according to Claim 22, in which the cosolvent is chosen from compounds that are at least partially or totally soluble in oxides of organic sulfides a), preferably chosen from carbonates, esters, ketones, amines, amides and alcohols, more preferably from alcohols, which are preferably saturated, preferably comprising from 1 to 10 carbon atoms, and more preferably still from methanol, ethanol, propanol, butanol, pentanol, ethylene glycol, propylene glycol and glycerol (1,2,3-propanetriol).

24. Composition according to Claim 22, comprising:
a) 80% by weight of DMSO;
b) from 10 ppm to 2%, preferably from 10 ppm to 1% by weight, relative to the total weight of the composition, of masking agent comprising:
- from 5% to 30% by weight of at least one monoester b1), chosen from isoamyl acetate, ethyl 2-methylbutyrate, isoamyl butyrate, phenylethyl acetate, ethyl caproate, benzyl acetate and hexyl acetate, and mixtures thereof;
- from 20% to 60% by weight of at least one diester and/or triester b2) chosen from ortho-phthalates, such as diethyl ortho-phthalate; citrates, such as triethyl citrate; malonates, such as diethyl malonate, and mixtures thereof;
- from 5% to 25% by weight of at least one alcohol, preferably of at least two alcohols, more preferably of at least three alcohols, as described previously in b3) ;
- from 1% to 10% by weight of at least one ketone, preferably at least two ketones, more preferably at least three ketones, as described previously in b4); and
- from 1% to 10% by weight of at least one, preferably at least two, and preferably a mixture of terpenes referenced above in b5); and
c) 20% by weight of glycerol.
